# EUROPÄISCHE PATENTSCHRIFT

(11) **EP 2 129 408 B1**
(45) Veröffentlichungstag und Bekanntmachung des Hinweises auf die Patenterteilung: **25.01.2017**
(21) Anmeldenummer: 07818335.7
(22) Anmeldetag: 21.09.2007
(51) Int. Cl.: A61L 31/10, A61L 31/16

(54) **BESCHICHTETES IMPLANTAT**
COATED IMPLANT
IMPLANT REVÊTU

(30) Priorität: 22.09.2006 DE 102006045272; 02.04.2007 DE 102007016151
(43) Veröffentlichungstag der Anmeldung: 09.12.2009
(73) Patentinhaber: Han, Bock-Sun, 52074 Aachen (DE)
(72) Erfinder: SELLIN, Lothar, 52074 Aachen (DE); HAN, Bock-Sun, 52074 Aachen (DE); AUTSCHBACH, Annelotte, 52074 Aachen (DE)
(74) Vertreter: Thiel, Christian
(86) Internationale Anmeldenummer: PCT/EP2007/008246
(87) Internationale Veröffentlichungsnummer: WO 2008/034627

(56) Entgegenhaltungen:
- WO-A-01/47572
- WO-A-2004/055153
- WO-A-2005/018665
- WO-A-2005/051229
- WO-A-2005/114720
- US-A- 6 140 127
- US-A1- 2005 268 722
- US-A1- 2006 121 639
- US-B1- 6 653 292
- AOKI J ET AL: "Endothelial Progenitor Cell Capture by Stents Coated With Antibody Against CD34" JOURNAL OF THE AMERICAN COLLEGE OF CARDIOLOGY, ELSEVIER, NEW YORK, NY, US, Bd. 45, Nr. 10, 17. Mai 2005 (2005-05-17), Seiten 1574-1579, XP004889595 ISSN: 0735-1097

## Beschreibung

Die Erfindung betrifft ein Implantat, das mit einer anspruchsgemäßen Beschichtung versehen ist. Das Implantat ist in erster Linie für das vaskuläre System bestimmt und insbesondere ein Stent, beispielsweise ein Coronarstent, kann aber auch anderweitig implantiert werden.

Stents werden mit Hilfe von endovaskulären Techniken in Blutgefäße eingesetzt, um Engpässe dauerhaft zu beseitigen, gegebenenfalls auch, um Fisteln oder Aneurysmen zu verschließen. In jedem Fall sollen sie das Gefäß, in das sie eingesetzt werden, durchgängig halten.

Andere Implantate, insbesondere im koronaren Bereich, dienen der Beseitigung von Defekten, beispielsweise Gefäßprothesen, Knochenersatz, Gelenkersatz, Herzklappen, Verschlüsse für den duktus botalli, wie auch Spezialimplantate zum Verschluss von Fisteln und zur Beseitigung von arteriovenösen Fehlbildungen. Im Folgenden wir die Erfindung vorwiegend anhand von Stents beschrieben.

Eine häufige Komplikation bei Stents ist die sogenannte Restenose, d.h. der Wiederverschluss des Gefäßes nach Implantation eines Stents. Die Restenose beruht auf der Proliferation von Zellen, insbesondere glatten Muskelzellen, die sich auf der Innenwand des Stents ansiedeln und dazu führen, dass sich das freie Lumen des Gefäßes im Stentbereich erneut verengt. Bei übermäßiger Zellanlagerung kann das an und für sich erwünschte Einwachsen des Stents dazu führen, dass es zu erneuten starken Gefäßverengungen im Stentbereich kommt, die insbesondere im Koronarbereich zu lebensbedrohlichen Situationen führen können.

Ein Grund für die Restenose ist die bei der Implantation eines Stents häufig auftretende Verletzung des Endotheliums mit entsprechenden Entzündungsreaktionen und einer Ausschüttung von Wachstumsfaktoren, die die Zellproliferation fördern. Die Verletzung der Gefäßwandung rühren vor allem daher, dass der Stent bei den herkömmlichen Ballon-Implantationstechniken mit erheblichen Drücken an und in die Gefäßwand gepresst wird, nicht nur um das Gefäß auf diese Weise auf ein zuträgliches Lumen aufzuweisen, sondern auch um den Stent in der Gefäßwand zu verankern und ihn so an seinem Implantationsort zu fixieren.

Derzeit wird davon ausgegangen, dass die Restenose maßgeblich durch die Umstände in den ersten Wochen nach der Implantation bestimmt wird. Mit der Abheilung der implantationsbedingten Wunden in der Gefäßwand klingen die entzündlichen Erscheinungen und die Ausschüttung der Wachstumsfaktoren ab. Die Zellproliferation kommt zum Stillstand. Allerdings sind die zu diesem Zeitpunkt bereits gebildeten Zellschichten auf der Innenwandung des Stents weiterhin Ansatzpunkt für erneute Ab- und Anlagerungen, die zu einem langfristigen Restenoseprozess führen können.

Für die durch den Stent verursachte Restenose gibt es zwei verschiedene Ursachen. Dies ist zum einen die Tatsache, dass in der ersten Zeit nach der Implantation die Oberfläche des Stents dem Blut direkt ausgesetzt ist und es aufgrund der nun vorhandenen Fremdoberfläche zu einer akuten Thrombose kommen kann, die das Blutgefäß wieder verschließt. Zum anderen sind dies die implantationsbedingten Gefäßwandverletzungen und die damit verbundenen entzündlichen Erscheinungen. Die hierbei ablaufenden Prozesse und die Ausschüttung von Wachstumsfaktoren führen zu einer verstärkten Proliferation der glatten Muskelzellen und damit schon kurzfristig zu einem erneuten Verschluss des Gefäßes aufgrund unkontrollierten Wachstums.

Nach einigen Wochen beginnt der Stent in das Gewebe des Blutgefäßes einzuwachsen. In der Regel führt dies dazu, dass der Stent vollständig von glatten Muskelzellen umhüllt wird und kein Kontakt mehr zum Blut hat. Das Einwachsen ist an und für sich erwünscht, die Vernarbung kann dabei aber zu stark ausgeprägt sein und dazu führen, dass nicht nur die Stentoberfläche bedeckt wird sondern der gesamte Innenraum des Stents zuwächst (Neointimahyperplasie).

In allen Fällen verbleiben implantierte Stents als Fremdkörper im Gewebe ohne dauerhaft darin integriert zu werden.

Es gibt eine Reihe von Ansätzen, um das Problem der Restenose zu lösen.

Auf der rein mechanischen Seite wird der Stent allseitig mit einer sehr sorgfältigen Politur geglättet, um die Anlagerung von Zellmaterial und die Verletzung des Endotheliums durch Rauhigkeiten und Grate zu unterbinden. Diese Methode bringt einigen Erfolg, wobei jedoch eine gewisse Restenoserate im Bereich von etwa 15% bislang nur schwer unterschritten werden kann.

Man hat vergeblich versucht, das Problem der thrombosebedingten Restenose durch die Ausstattung der Stents mit Heparin zu lösen, siehe J. Whöne et al., European Heart Journal 2001, 22, 1808-1816. Heparin adressiert als Antikoagulanz nur die thrombosebedingte Restenose und kann darüber hinaus nur in Lösung seine volle Wirkung entfalten. Hier hat sich eine medikamentöse Behandlung durchgesetzt.

Erste Versuche, die neointimale Proliferation durch Beschichten der Stents zu verhindern, blieben zumeist ohne durchschlagenden Erfolg. Weder eine Beschichtung mit Gold noch eine Siliciumcarbid- oder Carbonbeschichtung gab bislang eindeutige Ergebnisse.

Es wurde auch versucht, Stents mit proliferationshemmenden Medikamenten auszurüsten, um der Zellproliferation entgegenzuwirken. Bekannte Mittel hierfür sind Paclitaxel und Rapamycin. Damit ausgerüstete Stents haben derzeit eine günstigere Restenoserate als polierte Stents. Nichts desto weniger ist auch hier die Restenoserate verbesserungsbedürftig.

US-A-5,891,108 offenbart einen hohl ausgeformten Stent, welcher in seinem Innern pharmazeutische Wirkstoffe enthält, welche durch eine Vielzahl von Öffnungen im Stent freigesetzt werden. EP-A-1 127 582 beschreibt eine andere Variante eines Stents, der zur Aufnahme eines Wirkstoffes geeignet ist. Medikamentenhaltige Stentbeschichtungen sind beispielsweise aus WO 95/03036 A bekannt, wo insbesondere Paclitaxel enthaltende Beschichtungen beschrieben sind.

Derartig ausgerüstete Stents sind konstruktionsbedingt Wirkstoffreservoire, die den pharmazeutischen Wirkstoff punktuell in hoher Konzentration und über einen relativ langen Zeitraum freisetzen.

Während nicht proliferationshemmend ausgerüstete Stents innerhalb weniger Monate mit einer schützenden Zellschicht bedeckt werden, wirken proliferationshemmende Medikamente, beispielsweise Rapamycin und Paclitaxel, diesem Heilmechanismus entgegen. Dies führt dazu, dass die glatten Muskelzellen nicht mehr oder nur sehr verzögert in der Lage sind, den Stent zu umhüllen. Daher ist der Stent viel länger dem Blut ausgesetzt, was wieder vermehrt zu Gefäßverschlüssen durch Thrombosen führt, siehe F. Liestro, A. Colombo, "Late Acute Thrombosis after Paclitaxel Elluting Stent Implantation", Heart 2001, 86, 262-264. Die dadurch künstlich verlängerte Einheilungszeit stellt eine mehr oder weniger offene Wunde in der Gefäßwand dar, die leicht zu Gerinnseln und Thrombosen führen kann. So sind Thrombosen noch ein Jahr nach dem erfolgreichen und komplikationslosen Einsatz von medikamentbeschichteten Stents beobachtet worden, E. McFadden et al., Lancet 2004, 364, 1519-1521. Ferner scheinen nach jüngsten Erkenntnissen mit proliferationshemmenden Medikamenten beschichtete Implantate das Risiko von Herzanfällen deutlich zu erhöhen.

Hinzu kommt bei mit Medikamenten beschichteten Stents eine Tendenz zur ungleichmäßigen Abgabe des Wirkstoffs, die einer kontrollierten Einheilung des Stents entgegensteht.

Unter den jeweils herrschenden physiologischen Bedingungen kommt es häufig zu einer schubweisen oder verzögerten Freisetzung. Die verzögerte Freisetzung ist für den erwünschten Zweck nachteilig, da es insbesondere in den ersten Tagen nach der Implantation auf eine gleichmäßige Freisetzung des Wirkstoffs ankommt. Die schubweise Freisetzung ist unerwünscht, da es sich bei den eingesetzten Medikamenten um hochwirksame Systeme handelt, die in höheren Konzentrationen Schäden verursachen können.

Aus WO 2004/055153 A ist die Verwendung von Aptameren zur Beschichtung von Oberflächen zur Förderung der Adhäsion von biologischem Material bekannt. Bei den beschichteten Gegenständen kann es sich um Implantate handeln, darunter auch solche, die für das vaskuläre System bestimmt sind. Bei dem biologischem Material kann es sich beispielsweise um Stammzellen, Epithelzellen und dergleichen sowie deren Vorläuferzellen handeln. Die Aptamere sind an die Implantatoberfläche gebunden. Die Oberfläche, d. h. das Implantat kann dabei aus einem Kunststoffmaterial bestehen. Die Anbindung erfolgt auf photochemische Art und Weise.

Insbesondere bei Stents ist das Aufbringen von Kunststoffschichten problematisch, da durch das Aufkrimpen eines Stents auf einen üblichen Implantationsballon und die anschließende Expansion die Beschichtung einer erheblichen Belastung ausgesetzt wird. Insoweit sind zahlreiche Kunststoffmaterialien für solche Beschichtungszwecke nicht geeignet. Beispielhaft sei auf Acrylatmaterialien verwiesen. Zudem ist die Eignung von Kunststoffen für Beschichtungen im vaskulären System zumeist nur unzureichend untersucht. Wünschenswert wären hier Kunststoffmaterialien, die geeignet sind, die Ansiedlung von Epithelzellen auf der Oberfläche zu begünstigen.

Ein weiterer Ansatz stellt auf die Phosphorylcholinbeschichtung von Stents ab, siehe WO 01/01957 A. Hierbei wird Phosphorylcholin, eine Zellmembrankomponente der Erythrozyten, als Bestandteil einer nicht bioabbaubaren Polymerschicht auf dem Stent dazu verwandt, eine nichtthrombogene Oberfläche zu erzeugen. Der Wirkstoff, wird, abhängig vom Molekulargewicht, von der Beschichtung absorbiert oder an der Oberfläche festgehalten.

Bekannt geworden sind ferner spezielle Mikroproteine mit bis zu 40 Aminosäuren, die in der Lage sind, konformationell stabile dreidimensionale Strukturen einzunehmen, was sie zu vielseitig einsetzbaren Bindemoleküle macht. Beipiele für solche Mikroproteine sind Cystin-Knotenproteine (Krause et al., FEBS 2007, 274, 86-95).

Aufgabe der Erfindung ist die Bereitstellung von Implantaten, insbesondere Stents, die die Nachteile der bekannten medikamentösen Beschichtung vermeiden und eine zuverlässige und kontrollierte Einheilung gewährleisten, insbesondere aber die Ansiedlung von Epithelzellen auf der Oberfläche begünstigen.

Dieses Ziel wird mit einem Implantat, insbesondere Stent der eingangs genannten Art erreicht, das eine Amino-funktionalisierte Parylenschicht und ein anspruchsgemäßes Nukleotid-Aptamer aufweist, das eine spezifische Bindungsaffinität für CD 34-positive Zellen hat.

In der Regel handelt es sich dabei um eine Affinität für humane CD 34-positive Zellen.

Schicht bzw. Beschichtung im Sinne der Erfindung ist jede Art von Beschichtung, die auf das Implantat oberflächlich aufgebracht wird. Beschichtungen im Sinne der Erfindung sind die Amino-funktionalisierte Parylenschicht und die anspruchsgemäßen Nukleotid-Aptamere.

Als Schichten im Sinne der Offenbarung gelten auch modifizierte Oberflächen, wie Oxidschichten, hydroxylierte, aminierte oder anderweitig modifizierte Oberflächen, ebenso Schichten aus Kunststoffen oder anderen Materialien.

Endothelprogenitorzellen (EPC) sind CD 34-positive Zellen. Dies bedeutet, dass sie mit Oligonukleotiden oder Aptameren, die eine spezifische Bindungskapazität für CD 34-positive Zellen aufweisen, wechselwirken und eine Bindung eingehen. Beschichtungen, die auf CD 34-positive Zellen spezifisch reagierende Oligonukleotide gebunden enthalten, sind also in der Lage, Endothelprogenitorzellen aus dem Blutkreislauf zu binden und an der Oberfläche festzuhalten. Diese Endothelzellen können somit eine Endothelschicht auf der Implantatoberfläche generieren, die der Einwachsung des Stents in die Gefäßwand dienlich ist.

Erfindungsgemäß kommen Oligonukleotide, die weniger als 100 Basen aufweisen und vielfach auch als Aptamere bezeichnet werden zum Einsatz. Es handelt sich dabei um DNS-Oligonukleotide mit hoher Affinität für bestimmte Zielstrukturen. Solche Aptamere können mit einer sehr hohen und spezifischen Bindungsaffinität zu den unterschiedlichsten Targets hergestellt werden. Zu diesen Targets gehören beispielsweise Aminosäuren, Antikörper, Proteine, aber auch Zellen, insbesondere CD 34-positive Zellen.
Verfahren zur Generierung von Aptameren, die praktisch jedes beliebige Molekül molekular erkennen und hochspezifisch binden können, sind bekannt, beispielsweise die SELEX-Prozedur. Gleiches gilt für die Selektionierung von Aptameren für die selektive Proteinbindung und Inhibierung von deren Funktionen.

Offenbart sind auch auch Oligopeptide bzw. "Peptidaptamere", die die entsprechende Affinität zu CD 34-positiven Zellen haben.

Diese Peptide können durch einfache, dem Fachmann bekannte Techniken identifiziert und hergestellt werden.

Solche Oligopeptide sind beispielsweise sogenannte Cystin-Knoten-Mikroproteine, peptidische Biomoleküle mit 28 - 40 Aminosäuren. Sie weisen eine charakteristische Verknüpfung von 6 Cysteinen zu einem Cystin-Knoten und ein dreisträngiges antiparalleles Beta-Faltblatt auf. Aufgrund ihrer hohen konformationellen Stabilität können Mikroproteine durch Austausch einzelner oder Einfügen zusätzlicher Aminosäuren innerhalb exponierter Schleifen funktionalisiert werden, was sie zu therapeutisch einsetzbaren Bindemolekülen macht.

Erfindungsgemäße Nukleotid-Aptamere sind die folgenden, an und für sich bekannten Nukleotid-Sequenzen:
ccg ccg tgc ggg gta att tct ttt cca taa cga t (Seq. 1)
tcc tgc agc ttc ctg atg ct (Seq. 2)
gat tgc ctg acg tca tag ag (Seq. 3)
cgg cgg ctg acg tca gag ccg (Seq. 4)
ccg tcg ttt tgt cgt ttt gtc (Seq. 5).

Weitere geeignete Nukleotid-Aptamere sind die in der WO 2004/055153 A genannten, die hier ausdrücklich einbezogen werden.

In Frage kommen ferner die entsprechenden Spiegelaptamere.

Erfindungsgemäß werden folglich unter geeigneten Oligonukleotiden oder Aptameren solche verstanden, die an CD 34-positive Zellen binden können, wie auch ihre chemisch modifizierten Varianten mit gleichem Bindungsverhalten.

Es versteht sich, dass das hier beschriebene Prinzip nicht nur für Stents geeignet ist, sondern für jede Art von Implantaten, die von einer Anbindung von CD 34-positiven Zellen profitieren können, beispielsweise Herzklappen, Verschlusselemente für Aneurysmen oder Fisteln, Gefäßprothesen, Knochenund Gelenkersatz, Zahnimplantaten und dergleichen. Als Implantate im Sinne der Erfindung gelten auch temporäre Stents und Dilatationsballone, die erfindungsgemäß behandelt werden (temporäre Implantate).

Als Beschichtung für erfindungsgemäße Implantate kommt jede Art von Aminofunktionalisierter Parylenbeschichtung in Frage, die geeignet ist, die erfindungsgemäß zum Einsatz kommenden Oligonukleotide oder Aptamere festzuhalten. In Frage kommen insbesondere solche mit Parylen A und AM. Solche Beschichtungen haben beispielsweise eine Schichtdicke im Bereich von 2 µm bis 10 µm.

Untersuchungen haben gezeigt, dass mit einer Amino-funktionalisierte Parylenschicht ausgestattete Stents auch nach der Expansion eine intakte Oberfläche aufweisen, an die sich Epithelprogenitorzellen bereitwillig anlagern. Die angelagerten Zellen weisen eine natürliche Morphologie aus, was bei rein metallischen Stents nicht der Fall ist. Ist der Stent mit einem aminofunktionalisierten Parylen beschichtet, wird die Besiedlung der Stentoberfläche mit EPCs deutlich dichter und beschleunigt sich. Insoweit ergänzen sich die funktionalisierte Parylenbeschichtung und eine darauf aufgebrachte Aptamerbeschichtung hin zu einer schnell durch EPCs besiedelbaren Implantatoberfläche.

Da an Parylenschichten auch Proteine aus dem Blut anbinden, stellt es eine sehr gute Grundbeschichtung dar mit hoher Adhäsionskraft für Zellen, die sich dort in einer relativ natürlichen Umgebung absiedeln.

Beschichtungen mit Parylen A und AM seien hier an erster Stelle genannt, jedoch kommt jede andere Art von Aminofunktionalisierung in Frage, die geeignet ist, die hier zum Einsatz kommenden Aptamere kovalent oder anders (adhäsiv) zu binden.

Die Implantate werden auf übliche Art und Weise mit den in Frage kommenden Materialien beschichtet, insbesondere durch CVD-Niederdruck-Plasmaverfahren.

Geeignet sind auch Parylene mit Aminofunktionen, die durch eine nachträgliche Funktionalisierung auf an und für sich bekannte Art und Weise eingebracht wurden. Eine Funktionsdichte von 10¹⁰ bis 10¹⁵, insbesondere 10¹² bis 10¹⁴ Amino-Funktionen/cm² ist im allgemeinen ausreichend.

Im Ergebnis handelt es sich bei der Parylenbeschichtung der erfindungsgemäßen Implantate sowohl um einen Haftvermittler als auch um einen "Wirkstoff". Da die Oligonukleotide bzw. Aptamere nur unzureichend an die Metalloberfläche des Stents anbinden, werden Ankergruppen benötigt, die auf einem Material bereitgestellt werden, dass selbst gut auf der Implantatoberfläche haftet. Dies ist bei Parylen-Beschichtungungen gesichert. Die funktionalisierte Beschichtung dient als Träger für die Aptamere, die ihrerseits als Links für die EPCs dienen, gleichzeitig aber auch als "Andockstelle" für die EPCs.

Erfindungsgemäß können die Oligonukleotide bzw. Aptamere auch an eine weitere Schicht gebunden sein, beispielsweise an eine DLC-Schicht (Diamond-Like-Carbon) oder eine andere Kunststoffschicht. DLC-Schichten können beispielsweise durch Sputtern in eine Vakuumkammer mit Graphit als Substrat und dem Implantat als Target erzeugt werden. Soweit eine Funktionalisierung zum Binden der Oligonukleotide bzw. Aptamere erforderlich ist, kann diese auf an und für sich bekannte Art und Weise vorgenommen werden.

Die erfindungsgemäßen Implantate können zusätzlich eine hämokompatible Schicht aufweisen, die insbesondere als Grund- oder Basisschicht unmittelbar auf der Implantatoberfläche aufsitzt. Sie besitzen dann ggf. eine oder mehrere weitere Schichten, darunter mindestens eine weitere Schicht, an die die vorstehend beschriebenen Oligonukleotide bzw. Aptamere gebunden sind.

Als hämokompatible Schichten kommen insbesondere auch oberflächlich aufgebrachte Oxidschichten des Implantates in Frage, die beispielsweise durch oberflächliche Oxidation eines Metallstents aus Nitinol erhalten werden können. Die Implantatoberflächen können auch durch Hydroxilierung oder Aminierung so modifiziert werden, dass sie erfindungsgemäß Aptamere binden können.

Die hämokompatible Beschichtung, beispielsweise eines Stents, sorgt für eine weitere Verbesserung der Blutverträglichkeit, während die Aptamere für das schnelle Andocken von Endothelzellen und damit das schnelle Einwachsen des Stents in die Gefäßwand stehen. Die gleichmäßige Verteilung der Aptamere über die Gesamtoberfläche des Stents bewirkt das gleichmäßige und kontrollierte Anwachsen der Zellen, neben Endothelzellen auch von glatten Muskelzellen. Es findet somit eine rasche Besiedlung der Stentoberfläche und der Stentzwischenräume mit Zellen statt, was eine Verhinderung der Restenose mit sich bringt und das Risiko von Thrombosen, insbesondere nach einer kurzen Einheilungszeit stark vermindert. Tatsächlich findet das Andocken der EPCs innerhalb weniger Stunden statt, wie Versuche gezeigt haben.

Die hämokompatible Schicht kann auch adhäsiv oder kovalent gebunden an eine Polymermatrix, beispielsweise aus Polyacrylsäure, Polyvinylpyrolidon, Polyethylenglykol oder anderen medizinisch geeigneten Polymeren vorliegen. Sie bzw. diese können antithrombotisch oder antiproliferativ wirkende Wirkstoffe enthalten.

Zur Herstellung mehrfach beschichteter Implantate werden die einzelnen weiteren Schichten auf herkömmliche Art und Weise aufgebracht, beispielsweise mit Tauch- oder Sprühverfahren.

Es versteht sich, dass die erfindungsgemäßen Implantate auch mehrere Schichten aus hämokompatiblen Materialien und/oder Amino-funktionalisierten Parylenen und/oder Aptameren enthalten können.

Die hämokompatible Schicht kann auch in Form eines biolöslichen Polymers als äußere Schicht auf den Stent aufgebracht werden, so dass sich diese Schicht kurze Zeit nach der Einbringung in das aufgeweitete Gefäß auflöst oder abbaut. Derartige biolösliche Polymere sind an und für sich bekannt.

Die Offenbarung betrifft schließlich die Verwendung von Amino-funktionalisiertem Parylen-A als Beschichtungsmaterial für Implantate und insbesondere für vaskuläre Stents. Bevorzugt sind Parylen A und AM mit und ohne daran gebundenen Oligonukleotiden, Oligopeptiden bzw. Aptameren.

Es sei schließlich noch auf die Möglichkeit hingewiesen, die erfindungsgemäßen Implantate mit molekular geprägten Polymeren auszurüsten, d.h. mit einer Schicht, die eine spezifische Bindungsaffinität für CD 34-positive Zellen aufweist und molekular geprägte Polymere enthält. Diese Polymere können in Form einer Beschichtung vorliegen, aber auch in Form von Nanopartikeln, die allein oder zusätzlich auf die Implantatoberfläche aufgebracht werden.

Mit der Technologie des molekularen Prägens können synthetische Materialien für die molekulare Erkennung erzeugt werden, die in ihrer Affinität mit biologischen Systemen vergleichbar sind. Molekulares Prägen (molecular imprinting) ist eine Templat-Polymerisation, die künstliche molekulare Erkennungsstellen kreiert. Dazu werden die Zielmoleküle mit funktionellen Monomeren und Vernetzern gemischt und anschließend einer radikalischen Polymerisation unterworfen, die ein hoch vernetztes Polymer ausbildet. Die Zielmoleküle wirken dabei als Template - die Polymerisation findet um sie herum statt. Werden die Templatmoleküle durch Extraktion entfernt, verbleiben im Polymernetzwerk Hohlräume, welche die räumliche Anordnung funktioneller Gruppen abbilden. Durch dieses Einfrieren der Struktur entstehen spezifische Erkennungsstellen im Polymermaterial (C. Gruber-Traub et al., Polymer Preprints 2006, 47 (2), 901 - 902). Als Zielmoleküle dienen dabei die jeweils interessierenden Zielstrukturen, im vorliegenden Fall die Zielstrukturen der CD 34-positiven Zellen bzw. Endothelprogenitorzellen.

Als Implantate im Sinne der Erfindung gelten auch temporäre Implantate, beispielsweise temporäre Stents, die sich nach einer gewissen Verweildauer im Körper auflösen (Magnesiumstents) oder wieder entfernt werden. Hierzu gehören auch Dilatationsballone. Bei diesen Implantaten beruht die Funktionsweise auf einem Imprägnierungseffekt, d.h. die Beschichtung des Implantats wird beispielsweise auf die Gefäßwand übertragen und entfaltet dort ihre Wirkung.

## Patentansprüche

1. Implantat, insbesondere Implantat für das vaskuläre System, mit einer Beschichtung, die ein Nukleotid-Aptamer enthält, **dadurch gekennzeichnet, dass** das Nukleotid-Aptamer an eine Amino-funktionalisierte Parylenschicht, chemisch oder physikalisch gebunden ist, wobei das Nukleotid-Aptamer eine spezifische Bindungsaffinität für CD 34-positive Zellen hat und eine der folgenden Sequenzen aufweist:
ccg ccg tgc ggg gta att tct ttt cca taa cga t (Seq. 1)
tcc tgc agc ttc ctg atg ct (Seq. 2)
gat tgc ctg acg tca tag ag (Seq. 3)
cgg cgg ctg acg tca gag ccg (Seq. 4)
ccg tcg ttt tgt cgt ttt gtc (Seq. 5).

2. Implantat nach einem der vorstehenden Ansprüche, **dadurch gekennzeichnet, dass** die Amino-funktionalisierte Parylenschicht durch eine CVD- oder Plasma-Beschichtung aufgebracht ist.

3. Implantat nach einem der vorstehenden Ansprüche, **dadurch gekennzeichnet, dass** die Amino-funktionalisierte Parylenschicht eine Parylen-A-oder Parylen-AM-Schicht ist.

4. Implantat nach Anspruch 3, **dadurch gekennzeichnet, dass** die Funktionsdichte 10¹² bis 10¹⁴ Amino-Funktionen/cm² beträgt.

5. Implantat nach einem der Ansprüche 1 bis 4, **dadurch gekennzeichnet, dass** es zusätzlich eine hämokompatible Basisschicht aufweist.

6. Implantat nach Anspruch 5, **dadurch gekennzeichnet, dass** die Basisschicht eine Oxidschicht, eine durch Aminierung erhaltene Implantatoberfläche oder eine Kunststoffschicht ist.

7. Implantat nach einem der vorstehenden Ansprüche, **gekennzeichnet durch** eine biolösliche Deckschicht.

8. Stent nach einem der vorstehenden Ansprüche.

9. Coronarstent nach Anspruch 8.

## Claims

1. Implant, especially implant for the vascular system, provided with a coating comprising a nucleotide-aptamer, **characterized in that** the nucleotide-aptamer is chemically or physically bonded to an amino-functionalized parylene coat, the nucleotide-aptamer having a specific bonding affinity with CD34-positive cells and the nucleotide-aptamer comprises one of the following sequences:
ccg ccg tgc ggg gta att tct ttt cca taa cga t (Seq. 1)
tcc tgc agc ttc ctg atg ct (Seq. 2)
gat tgc ctg acg tca tag ag (Seq. 3)
cgg cgg ctg acg tca gag ccg (Seq. 4)
ccg tcg ttt tgt cgt ttt gtc (Seq. 5).

2. Implant according to claim 1, **characterized in that** the amino-functionalized parylene coat is applied by means of a CVD or plasma coating process.

3. Implant according to any one of the above claims, **characterized in that** the amino-functionalized parylene coat is a parylene A or parylene AM coat.

4. Implant according to claim 3, **characterized in that** the function density amounts to 10¹² to 10¹⁴ amino functions per cm².

5. Implant according to any one of claims 1 to 4, **characterized in that** it additionally comprises a haemocompatible basic layer.

6. Implant according to claim 5, **characterized in that** the basic layer is an oxide layer, an implant surface obtained through amination, or a plastic layer.

7. Implant according to any one of the above claims, **characterized by** a biosoluble final coat.

8. Stent according to any one of the above claims.

9. Coronary stent according to claim 8.

## Revendications

1. Implant, en particulier implant pour le système vasculaire, avec un revêtement contenant un nucléotide aptamère, **caractérisé en ce que** le nucléotide aptamère est lié physiquement ou chimiquement à une couche de parylène amino-fonctionnalisée, le nucléotide aptamère ayant une affinité de liaison spécifique pour les cellules CD34+ et présentant une des séquences suivantes :
ccg ccg tgc ggg gta att tct ttt cca taa cga t (seq. 1)
tcc tgc agc ttc ctg atg ct (seq. 2)
gat tgc ctg acg tca tag ag (seq. 3)
cgg cgg ctg acg tca gag ccg (seq. 4)
ccg tcg ttt tgt cgt ttt gtc (seq. 5).

2. Implant selon l'une des revendications précédentes, **caractérisé en ce que** la couche de parylène amino-fonctionnalisée est appliquée par revêtement CVD ou plasma.

3. Implant selon l'une des revendications précédentes, **caractérisé en ce que** la couche de parylène amino-fonctionnalisée est une couche de parylène A ou une couche de parylène AM.

4. Implant selon la revendication 3, **caractérisé en ce que** la densité fonctionnelle est de 10¹² à 10¹⁴ fonctions amino / cm².

5. Implant selon une des revendications 1 à 4, **caractérisé en ce qu'**il présente en plus une couche de base hémocompatible.

6. Implant selon la revendication 5, **caractérisé en ce que** la couche de base est une couche d'oxyde, une surface d'implant obtenue par amination ou une couche de plastique.

7. Implant selon l'une des revendications précédentes, **caractérisé par** une couche de couverture biosoluble.

8. Stent selon une des revendications précédentes.

9. Stent coronaire selon la revendication 8.
